(19) Europäisches Patentamt
European Patent Office
Office européen des brevets

(11) EP 0 657 439 B1

(12) **EUROPEAN PATENT SPECIFICATION**

(45) Date of publication and mention of the grant of the patent: **16.07.1997 Bulletin 1997/29**

(51) Int. Cl.$^6$: **C07D 263/20**, A61K 31/42

(21) Application number: **94118198.4**

(22) Date of filing: **18.11.1994**

(54) **Process improvement in the synthesis of [R-(R*,R*)]-5-(3-chlorophenyl)-3-(2-(3,4-dihydroxyphenyl)-1-methylethyl)-2-oxazolididone**

Verbesserung bei der Herstellung [R-(R*, R*)]-5-(3-chlorophenyl)-3-(2-(3,4-dihydroxyphenyl)-1-methylethyl-2-Oxazolidinone

Amélioration de la production de l'[R-(R*,R*)]-5-(3-chlorophenyle)-3-(2-(3,4-dihydroxyphenyle)-1-methyleethyle-2-oxazolidinone

(84) Designated Contracting States:
**AT BE CH DE DK ES FR GB GR IE IT LI LU NL PT SE**
Designated Extension States:
**SI**

(30) Priority: **09.12.1993 US 164524**
**20.09.1994 US 309303**

(43) Date of publication of application: **14.06.1995 Bulletin 1995/24**

(73) Proprietor: **American Cyanamid Company**
**Madison, New Jersey 07940-0874 (US)**

(72) Inventors:
- **Cain, William Thomas**
  **New York (US)**
- **Cruz, Kelvin**
  **New Windsor, New York (US)**
- **McCoy, Kevin Michael**
  **Hoboken, New Jersey (US)**
- **Mohan, Arthur G.**
  **Somerville, New Jersey (US)**
- **Blum, David Michael**
  **Saddle River, New Jersey (US)**

(74) Representative: **Wileman, David Francis Dr.**
**c/o Patent Department**
**Wyeth Laboratories**
**Huntercombe Lane South**
**Taplow Maidenhead Berkshire SL6 OPH (GB)**

(56) References cited:
**US-A- 5 061 727**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 657 439 B1

Printed by Rank Xerox (UK) Business Services
2.14.11/3.4

## Description

Field of the Invention

The invention is a process improvement for producing [R-(R*, R*)]-5-(3-chlorophenyl)-3-[2-(3,4-dihydroxyphenyl)-1-methylethyl]-2-oxazolidinone which is an intermediate useful in making compounds that have antidiabetic and/or antihyperglycemic and/or antiobesity properties in mammals.

Background of Invention

United States Patent No. 5,061,727 teaches one method of making the desired oxazolidinone product. The oxazolidinone is isolated after an aqueous quench, extraction with an organic solvent, concentration, trituration with methyl alcohol and recrystallization in 75% overall yield and 80-90% purity.

Summary of the Invention

The invention is a one step process improvement for synthesizing a compound of Formula 1:

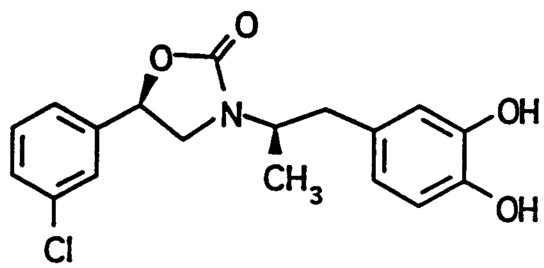

1

which comprises:

1.) reacting a compound of the formula:

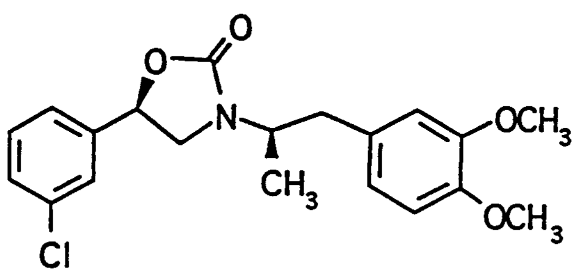

with boron tribromide in methylene chloride, under argon or nitrogen, at -15 to +20°C, preferably -10 to +15°C for 0.5-2 hours;
2.) quenching the reaction mixture, at -5 to 10°C, preferably 0 to 10°C with methyl alcohol;
3.) concentrating the reaction mixture, by distillation of methylene chloride;
4.) adding room temperature water to reaction;
5.) and collecting the precipitated crystalline product of formula 1.

The advantages of this process over the cited literature are higher yields of product; higher purity of product; the elimination of a separate purification step; and the prevention of the formation of gummy solids, which are undesirable in kilogram scale reactions.

The process if further improved by the simultaneous addition of boron tribromide and a solution of [R-(R*,R*)]-5-(3-chlorophenyl-3-[2-(3,4-dimethoxyphenyl)-1-methylethyl]-2-oxazolidinone in methylene chloride to methylene chloride while maintaining a constant ratio of 2.10:1.00 of boron tribromide to the substrate [R-(R*,R*)]-5-(3-chlorophenyl-3-[2-(3,4-dimethoxyphenyl)-1-methylethyl]-2-oxazolidinone at a temperature of -5°C to 10°C gives [R-(R*, R*)]-5-(3-chlorophenyl-3-[2-(3,4-hydroxyoxyphenyl)-1-methylethyl]-2-oxazolidinone in an isolated yield of 86.7% and a purity of 98.7%.

## DETAILED DESCRIPTION OF THE INVENTION

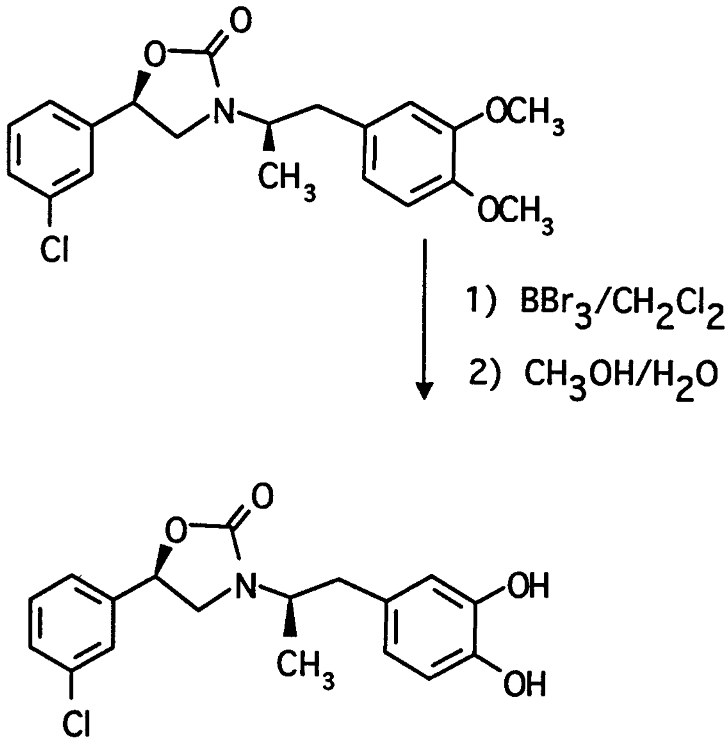


1

Referring to Scheme 1, [R-(R*,R*)]-5-(3-chlorophenyl)-3-[2-(3,4-dimethyloxyphenyl)-1-methylethyl]-2-oxazolidinone, obtained by literature procedures previously cited, is dissolved in methylene chloride, under argon or nitrogen at 0-5°C and boron tribromide is added, dropwise, to the reaction mixture. The progress of the reaction is monitored by thin layer chromatography or high pressure liquid chromatography. When the reaction is complete, it is quenched slowly with methyl alcohol at 0-10°C, stirred at room temperature for 1-4 hours, and the excess solvent is removed by distillation. The mixture is cooled to room temperature. Water is added, dropwise, and the reaction mixture is stirred at room temperature for 2-72 hours. The resultant slurry is cooled to 5-10°C and stirred for 1.5 hours. The precipitate is collected, washed with water and dried to give the desired product, 1', in 88-94% yield and >95% wt/wt purity.

The advantages of this process over the cited literature are:

1.) higher yields of product (90 versus 75%);
2.) higher purities of product (>95 versus 80-90% wt/wt)
3.) the elimination of a separate purification step;
4.) and the prevention of the formation of gummy solids, which are undesirable in kilogram scale reactions.

This invention will be described in greater detail in conjunction with the following example.

### Example 1

[R-(R*,R*)]-5-(3-Chlorophenyl)-3-[2-(3,4-dihydroxyphenyl)-1-methylethyl]-2-oxazolidinone

Twenty grams of [R-(R*,R*)]-5-(3-chlorophenyl)-3-[2-(3,4-dimethyloxyphenyl)-1-methylethyl]-2-oxazolidinone, prepared by the procedure described in U. S. Patent No. 5.061,727, is dissolved in 100 ml of methylene chloride and cooled

to 5°C under a stream of argon. Ten ml of boron tribromide in 10 ml of methylene chloride is added dropwise over 25 minutes. The reaction mixture is stirred for 20 minutes at 0-5°C. One hundred and fifty ml of methyl alcohol is added over 40 minutes and the reaction is allowed to warm to room temperature over 1 hour. The solvent is removed by distillation (pot temperature 90°C) leaving an oil in the bottom of the flask. The reaction flask is charged with 36.9 g of methyl alcohol, cooled to room temperature and 148 g of water is added over 1.5 hours. The reaction is stirred at room temperature for 72 hours, followed by cooling to 5°C and stirring at this temperature for 1 hour. The resulting precipitate is collected, rinsed with water, and dried to give 17.1 g of the desired product (Yield 92.7% theory).
LC Purity >95% wt/wt.

| Calculated for $C_{18}H_{18}ClNO_4$: | | | | | |
|---|---|---|---|---|---|
| Theory: | C = 62.16; | H = 5.22; | N = 4.03; | B = 0.00; | Br = 0.00 |
| Found: | C = 62.05; | H = 5.25; | N = 3.96; | B = 0.02; | Br = 0.36 |

$[\alpha]_D^{26}$ = -24±1, C = 1.1%

PILOT PLANT RUN

Raw Materials:

| | |
|---|---|
| 9.9 kg | [R-(R*,R*)]-5-(3-chlorophenyl)-3-[2-(3,4-dimethyloxyphenyl)-1-methylethyl]-2-oxazolidinone |
| 69.0 kg | Methylene chloride |
| 14.0 kg | Boron tribromide |
| 59.0 kg | Methanol |
| 136.0 kg | Deionized water |

Equipment Set-up:

1 50 gallon (189.25 liter) kettle
1 100 gallon (378.5 liter) kettle
Distillation set-up between the kettles;
nitrogen purge valves;
Glycol to the condenser and jacket of 50 gallon (189.25 liter) kettle;
Water to the jacket of the 100 gallon (378.5 liter) kettle.

Procedure:

A 100% nitrogen purge rate is set up between the 2 kettles for 5 minutes, then the rate is reduced to 30-35%. Methylene chloride, 66.6 kg is charged into the 50 gallon (189.25 liter) kettle. Agitation at 75 RPM is started, 9.9 kg of [R-(R*,R*)]-5-(3-chlorophenyl)-3-[2-3,4-dimethyloxyphenyl)-1-methylethyl]-2-oxazolidinone is added and the contents of the kettle are cooled to 0-5°C. Boron tribromide, 14.0 kg, is charged into the reaction kettle at a set rate of 70 ml/min using a Cole-Parmer Teflon metering pump. The temperature is maintained at 0-5°C by adjusting the feed rate. After the completion of the addition, the nitrogen purge is stopped and the reaction is stirred for 60 minutes. The lines and metering pump are flushed out with 3 kg of methylene chloride. At the end of 1 hour, a 5 ml aliquot is taken to check the reaction progress. Thin layer chromatography indicates the completion of the reaction.

Methyl alcohol, 59-kg, is charged into the 50 gallon (189.25 liter) kettle over a period of 1 hour, while maintaining the temperature at 0-10°C by adjusting the flow rate of methyl alcohol. Distillation of the solvents is started by using hot water at about 50-60°C on the jacket of the 50 gallon (189.25 liter) kettle. Once the methylene chloride is removed, increase the jacket temperature to 70-80°C to distill the methyl alcohol. Distill until the volume is 30-35 liters collecting the distillate in the 100 gallon (378.5 liter) kettle. Cool the contents of the 50 gallon (189.25 liter) kettle to 20-25°C using water. Add 100 kg of deionized water to the 50 gallon (189.25 liter) kettle over a 2 hour period using a metering pump. Cool the batch to 0-5°C over 30-40 minutes using glycol on the jacket. Maintain the 0-5°C temperature overnight. Filter the batch on a 30 inch (0.762 m) nutshell fitted with polypropylene cloth. Wash the cake twice with 18 kg of deionized water that has been cooled to 0-4°C. Harvest the cake into poly-lined fiberglass trays and dry in a vacuum oven at 30-35°C until constant weight.
Yield 8,154 g (89.1% of theory), off white crystals.
LC Purity 96.2% wt/wt and 96.2% area.

| Calculated for $C_{18}H_{18}ClNO_4C$: | | | | | | |
|---|---|---|---|---|---|---|
| Theory: | C=62.16; | H=5.2; | N=4.03; | Cl=10.19; | B=0.00; | Br=0.00; |
| Found: | C=61.28; | H=5.10; | N=3.96; | Cl=10.09; | B=0.02; | Br=1.42. |

$[\alpha]_D^{26}$ = -24± 1, c=1.2%
$^1$H NMR(DMSO):δ 7.4-6.3(m,7H); 5.50(dd,5H,J=6.3 and 9.OHz); 4.02(m,1H); 3.89(t,1H,J=9.1Hz); 3.5-3.3(bs,2H); 3.29(dd,1H,J=6.3 and 9.OHz); 2.6-2.4(m,2H); 1.14(d,3H,J=6.7Hz).
$^{13}$CNMR(DMSO): δ 17.5, 39.5, 46.8, 49.8, 73.0, 115.5, 116.2, 119.6, 124.6, 126.1, 128.5, 129, 130.7, 133.4, 142.2, 143.8 145.2, 156.4.
IR(KBr): 3411, 2977, 1724, 1606 $cm^{-1}$.

The process of Scheme I is further improved wherein [R-(R*,R*)]-5-(3-chlorophenyl-3-[2-(3,4-dimethoxyphenyl)-1-methylethyl]-2-oxazolidinone prepared using the synthetic procedures described in U.S. 5,061,727, is dissolved in methylene chloride and added simultaneously along with boron tribromide to methylene chloride, under an inert gas while keeping the temperature at -5°C to 10°C to give after quenching with methyl alcohol [R-(R*,R*)]-5-(3-chlorophenyl-3-[2-(3,4-dihydroxyphenyl)-1-methylethyl]-2-oxazolidinone. In accordance with the reaction conditions, the ratio of boron tribromide to [R-(R*,R*)]-5-(3-chlorophenyl-3-[2-(3,4-dimethoxyphenyl)-1-methylethyl]-2-oxazolidinone is maintained constant at 2.10:1.00 during the simultaneous addition of the reactants. The progress of the reaction can be followed by taking aliquots of the reaction mixture, quenching with methyl alcohol and analyzing by high pressure liquid chromatography (HPLC).

After complete addition of both the boron tribromide and [R-(R*,R*)]-5-(3-chlorophenyl-3-[2-(3,4-dimethoxyphenyl)-1-methylethyl]-2-oxazolidinone, the reaction mixture is maintained at 0-5°C for 10 minutes and methanol is slowly added at 0-10°C to destroy excess reagent. Evaporation of the solvents and adding water to the concentrate with water gives the desired [R-(R*,R*)]-5-(3-chlorophenyl-3-[2-(3,4-dihydroxyphenyl)-1- methylethyl]-2-oxazolidinone as a crystalline solid in high yield and purity.

## Claims

**1.** A process for making a compound of formula 1:

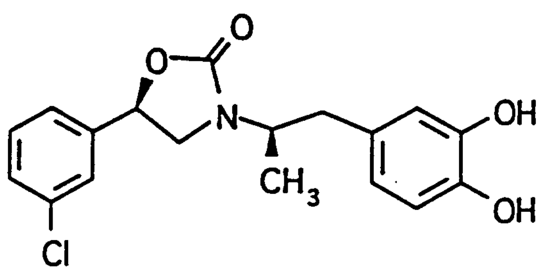

1

which comprises:

a) reacting, at -15° to +20°, [R(R*,R*)]-5-(3-chlorophenyl)-3-[2-(3,4-dimethyoxyphenyl)-1-methylethyl]-2-oxazolidinone with boron tribromide in methylene chloride, under argon for from 0.5 to 2 hours;
b) quenching the reaction mixture of -5 to 10°C with methyl alcohol;
c) concentrating the reaction mixture, by distillation of methylene chloride;
d) adding room temperature water to the reaction residue; and
e) collecting the precipitated crystalline product of formula 1.

**2.** The process of claim 1 wherein the reaction is at 0 to 5°C and the reaction mixture is quenched at 0 to 10°C.

**3.** The process of claim 1 the reaction comprises adding:

(a) a of methylene chloride solution of [R(R*,R*)]-5-(3-chlorophenyl-3-[2-(3,4-dimethoxyphenyl)-1-methylethyl]-2-oxazolidinone to methylene chloride while simultaneously adding boron tribromide at -5° to 10°C under inert gas at a rate so the ratio of boron tribromide to substrate is 2.10:1.00;
(b) quenching the reaction mixture with methyl alcohol at -5° to 10°C;
(c) concentrating the reaction mixture by distillation of the methylene chloride and a portion of the methyl alcohol;
(d) adding water to the reaction mixture;
(e) collecting the product by filtration and drying.

## Patentansprüche

1. Verfahren zur Herstellung einer Verbindung der Formel

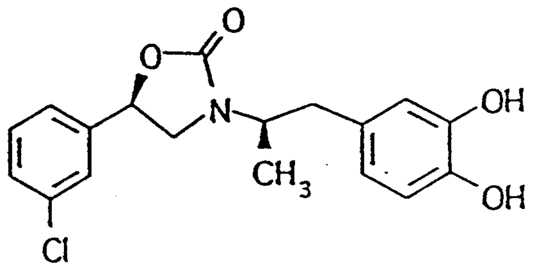

(1),

welches umfaßt:

a) Umsetzen, bei -15°C bis +20°C, von [R(R*,R*)]-5-(3-Chlorphenyl)-3-[2-(3,4-dimethoxyphenyl)-1-methylethyl]-2-oxazolidinon mit Bortribromid in Methylenchlorid, unter Argon während 0,5 bis 2 Stunden;
b) Abschrecken der Reaktionsmischung bei -5°C bis 10°C mit Methylalkohol;
c) Konzentrieren der Reaktionsmischung, durch Destillation von Methylenchlorid;
d) Zusetzen von Wasser bei Raumtemperatur zum Reaktionsrückstand; und
e) Sammeln des ausgefällten kristallinen Produkts der Formel (1).

2. Verfahren nach Anspruch 1, bei welchem die Reaktion bei 0°C bis 5°C stattfindet, und die Reaktionsmischung bei 0°C bis 10°C abgeschreckt wird.

3. Verfahren nach Anspruch 1, bei welchem die Reaktion das Zusetzen umfaßt von:

(a) einer Methylenchlorid-Lösung von [R(R*,R*)]-5-(3-Chlorphenyl)-3-[2-(3,4-dimethoxyphenyl)-1-methylethyl]-2-oxazolidinon zu Methylenchlorid, während gleichzeitig Bortribromid bei -5°C bis 10°C unter Inertgas bei einer Rate zugesetzt wird, so daß das Verhältnis von Bortribromid zu Substrat 2,10:1,00 beträgt;
(b) Abschrecken der Reaktionsmischung mit Methylalkohol bei -5°C bis 10°C;
(c) Konzentrieren der Reaktionsmischung durch Destillation des Methylenchlorids und eines Teils des Methylalkohols;
(d) Zusetzen von Wasser zur Reaktionsmischung;
(e) Sammeln des Produkts durch Filtration und Trocknung.

## Revendications

1. Procédé de préparation d'un composé de formule I :

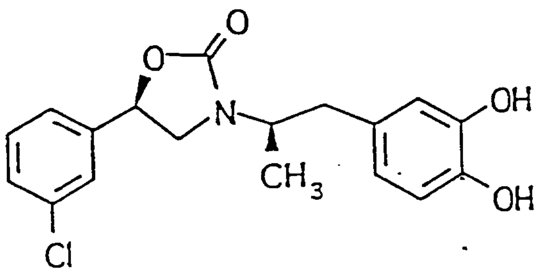

qui comprend :

a) la réaction, à -15° à +20°, de [R-(R*,R*)]-5-(3-chlorophényl)-3-[2-(3,4-diméthyloxyphényl)-1-méthyléthyl]-2-oxazolidinone avec du tribromure de bore dans du chlorure de méthylène, sous argon pendant 0,5-2 heures;
b) le traitement de la réaction, à -5° à +10°C avec de l'alcool méthylique;
c) la concentration du mélange de réaction, par distillation du chlorure de méthylène;
d) l'addition d'eau à la réaction à température ambiante; et
e) la récolte du produit cristallin précipité de formule I.

**2.** Procédé selon la revendication 1, dans lequel la réaction est effectuée à 0 à 5°C et le mélange réactionnel est traité à 0 à 10°C.

**3.** Procédé selon la revendication 1, dans lequel la réaction comprend :

(a) l'addition d'une solution de chlorure de méthylène de [R-(R*,R*)]-5-(3-chlorophényl)-3-[2-(3,4-diméthyloxyphényl)-1-méthyléthyl]-2-oxazolidinone au chlorure de méthylène tout en ajoutant simultanément le tribromure de bore à -5° à 10°C sous un gaz inerte à un taux tel que le rapport du tribromure de bore au substrat soit de 2,10:1,00;
(b) le traitement de la réaction avec de l'alcool méthylique à -5° à 10°C;
(c) la concentration du mélange de réaction par distillation du chlorure de méthylène et une partie de l'alcool méthylique;
(d) l'addition d'eau au mélange de réaction;
(e) la récolte du produit par filtration et séchage.